# EUROPÄISCHE PATENTSCHRIFT

(11) **EP 3 052 934 B1**
(45) Veröffentlichungstag und Bekanntmachung des Hinweises auf die Patenterteilung: **16.08.2017**
(21) Anmeldenummer: 14780834.9
(22) Anmeldetag: 01.10.2014
(51) Int. Cl.: G01N 33/49, A61B 5/157, B01L 3/00, G01N 21/00

(54) **VERFAHREN ZUM ERKENNEN EINES ZUSTANDS EINER PROBE, VORRICHTUNG ZUM ANALYSIEREN VON PROBEN UND LABORAUTOMATISIERUNGSSYSTEM**
METHOD FOR RECOGNISING A STATE OF A SAMPLE, DEVICE FOR ANALYSING SAMPLES AND LABORATORY AUTOMATION SYSTEM
PROCÉDÉ DE RECONNAISSANCE D'UN ÉTAT D'UN ÉCHANTILLON, DISPOSITIF D'ANALYSE DES ÉCHANTILLONS ET SYSTÈME DE LABORATOIRE AUTOMATIQUE

(30) Priorität: 01.10.2013 DE 102013219972
(43) Veröffentlichungstag der Anmeldung: 10.08.2016
(73) Patentinhaber: Roche Diagnostics GmbH, 68305 Mannheim (DE); F. Hoffmann-La Roche AG, 4070 Basel (CH)
(72) Erfinder: MÜLLER, Steffen, 74078 Heilbronn (DE)
(74) Vertreter: Patentanwälte Ruff, Wilhelm, Beier, Dauster & Partner mbB
(86) Internationale Anmeldenummer: PCT/EP2014/071061
(87) Internationale Veröffentlichungsnummer: WO 2015/049298

(56) Entgegenhaltungen:
- WO-A1-03/060484
- WO-A1-2011/019576
- WO-A1-2012/010787
- WO-A2-2005/003738

## Beschreibung

### Anwendungsgebiet und Stand der Technik

Die Erfindung betrifft ein Verfahren zum Erkennen eines Zustands aus einer vorgegebenen Menge von Zuständen einer Probe, insbesondere einer Blutprobe, welche sich in einem sich entlang einer Längsrichtung erstreckenden Probenröhrchen befindet, wobei die Menge von Zuständen einen nicht-gemischten Zustand und einen gemischten Zustand aufweist.

Proben, insbesondere medizinische Proben und darunter insbesondere Blutproben, werden häufig in gängigen Probenröhrchen abgefüllt, um sie Analysen zu unterziehen. Bei einer solchen Analyse kann beispielsweise das Vorhandensein einer Krankheit oder eine Alkoholkonzentration im Blut bestimmt werden. Typischerweise ist es bei vielen Analysen notwendig, einzelne Bestandteile einer solchen Probe vor der Analyse aufzutrennen. Dabei entstehen typischerweise in den Probenröhrchen mehrere Phasen unterschiedlicher Phasentypen.

Folgende Phasen, genauer Phasentypen, sind bei Blutproben beispielsweise bekannt:
- Luft: ein nicht mit Flüssigkeit oder festen Bestandteilen ausgefüllter Bereich, typischerweise am oberen Ende eines Probenröhrchens.
- Vollblut: nicht aufgetrenntes Blut mit allen Bestandteilen, wie es einem Menschen oder einem Tier entnommen wurde.
- Blutplasma bzw. Serum: flüssiger Anteil des Bluts, der als Überstand übrig bleibt, wenn aus einer Blutprobe die zellulären Bestandteile abgetrennt wurden.
- Blutkuchen: zelluläre Bestandteile von Blut, insbesondere rote Blutkörperchen, Blutplättchen und weiße Blutkörperchen.
- Gel: ein zur Verbesserung der Auftrennung in das Probenröhrchen zusätzlich zur Blutprobe eingebrachter Stoff, welcher im sichtbaren Spektrum weitgehend durchsichtig ist. Das Gel wird typischerweise bereits vom Hersteller des Probenröhrchens in das Probenröhrchen eingefüllt und befindet sich somit vor etwaig auszuführenden Trennschritten am unteren Rand bzw. Ende des Probenröhrchens.

Nach dem Einfüllen einer Blutprobe in ein Probenröhrchen befindet sich die Probe zunächst in einem gemischten Zustand, in welchem alle Bestandteile des menschlichen oder tierischen Bluts in einer einzigen homogenen Phase enthalten sind. Dies gilt zumindest solange, wie noch keine signifikante Blutsenkung eingesetzt hat. Vor dem Analysieren soll die Probe nun in einen nicht-gemischten Zustand überführt werden, in welchem die einzelnen Bestandteile des Bluts in getrennten Phasen vorliegen. Hierzu wird typischerweise die Probe zentrifugiert, was mittels einer üblichen Laborzentrifuge erfolgen kann. In diesem typischen Fall kann somit der gemischte Zustand als unzentrifugierter Zustand bezeichnet werden, und der nicht-gemischte Zustand kann als zentrifugierter Zustand bezeichnet werden.

Es sei verstanden, dass im Rahmen dieser Anmeldung häufig die Begriffe eines unzentrifugierten Zustands oder eines zentrifugierten Zustands verwendet werden, da es sich beim Verfahren des Zentrifugierens um das häufigste Verfahren zum Auftrennen einer Probe handelt. Grundsätzlich können jedoch auch andere Trennverfahren angewandt werden, und die Ergebnisse dieser Trennverfahren sollen durch den Begriff des zentrifugierten Zustands mit umfasst sein. Ebenso soll durch den Begriff des unzentrifugierten Zustands grundsätzlich ein Zustand bezeichnet sein, in welchem sich eine Probe ohne einen aktiv durchgeführten Trennungsschritt befindet, unabhängig von dem vorgesehenen Trennungsverfahren.

Wenn eine an der Probe durchzuführende Analyse einen nicht-gemischten Zustand erfordert, würde das Analysieren einer Probe im gemischten Zustand zumindest zu einem verfälschten Ergebnis der Analyse führen. Außerdem können Verstopfungen von Leitungen oder Beschädigungen am Analysegerät die Folge sein. Es sollten deshalb nur Proben im nicht-gemischten Zustand analysiert werden.

Bislang war es üblich, dass vor dem Analyseschritt manuell überprüft wird, ob sich jede einzelne Probe im nicht-gemischten Zustand befindet. Hierzu muss ein Mensch die jeweilige Probe betrachten und anhand der sichtbaren Phasentypen erkennen, ob sich die Probe im nicht-gemischten Zustand befindet. Dies ist einerseits zeitaufwändig, andererseits besteht auch die Gefahr menschlichen Versagens, so dass eine Probe im gemischten Zustand unter Umständen beispielsweise aufgrund von Unaufmerksamkeit nicht rechtzeitig identifiziert wird.

Die Erfindung betrifft des Weiteren eine Vorrichtung zum Analysieren von Proben, insbesondere Blutproben, welche in Probenröhrchen enthalten sind. Bei Verwendung einer solchen Vorrichtung können die bereits weiter oben erwähnten Analysen durchgeführt werden, wobei die bereits beschriebenen Probleme auftreten können.

Des Weiteren betrifft die Erfindung ein Laborautomatisierungssystem mit einer Vorrichtung zum Analysieren von Proben, wobei bei einem solchen Laborautomatisierungssystem ebenfalls die bereits beschriebenen Probleme bei der Vorrichtung zum Analysieren von Proben auftreten können.

Die WO 2011/019576 A1 zeigt ein Verfahren und eine Vorrichtung zum Analysieren von Blutproben, die in Probenröhrchen enthalten sind.

Die WO 2012/010787 A1 zeigt eine Vorrichtung und ein Verfahren zum automatisierten Detektieren von Phasen innerhalb eines Röhrchens, wobei das Detektieren auf Lichtreflexion basiert.

Die WO 2005/003738 A2 zeigt eine Verarbeitungseinheit für Blutspenden.

Die WO 03/060484 A1 zeigt eine Anordnung und ein Verfahren zur Analyse von Körperflüssigkeiten basierend auf Bildverarbeitung.

### Aufgabe und Lösung

Es ist deshalb eine Aufgabe der Erfindung, ein Verfahren zum Erkennen eines Zustands einer Probe bereitzustellen, welches eine automatisierte Erkennung eines Zustands erlaubt. Es ist des Weiteren eine Aufgabe der Erfindung, eine Vorrichtung zum Analysieren von Proben bereitzustellen, welche ein solches Verfahren anwendet. Außerdem ist es eine Aufgabe der Erfindung, ein Laborautomatisierungssystem mit einer solchen Vorrichtung bereitzustellen.

Dies wird erfindungsgemäß durch ein Verfahren nach Anspruch 1, eine Vorrichtung nach Anspruch 12 sowie ein Laborautomatisierungssystem nach Anspruch 14 erreicht. Vorteilhafte Ausgestaltungen können beispielsweise den jeweiligen Unteransprüchen entnommen werden. Der Inhalt der Ansprüche wird hiermit durch ausdrückliche Inbezugnahme zum Inhalt der Beschreibung gemacht.

Die Erfindung betrifft ein Verfahren zum automatisierten Erkennen eines Zustands aus einer vorgegebenen Menge von Zuständen einer Probe, insbesondere einer Blutprobe, mittels einer Vorrichtung. Die Menge von Zuständen weist einen nicht-gemischten Zustand und einen gemischten Zustand auf. Die Probe befindet sich in einem entlang einer Längsrichtung erstreckenden Probenröhrchen.

Das Verfahren weist folgende Schritte auf:
- Ermitteln zumindest einer Eigenschaft der Probe an einer Mehrzahl von unterschiedlichen Positionen entlang der Längsrichtung,
- Ermitteln mindestens eines Phasentyps einer Phase der Probe in dem Probenröhrchen anhand der ermittelten Eigenschaft, und
- Ermitteln des Zustands der Probe in Abhängigkeit von dem ermittelten Phasentyp.

Mittels des erfindungsgemäßen Verfahrens ist es möglich, die bislang manuell durchzuführende Erkennung unterschiedlicher Zustände einer Probe, welche sich vor einem Analyseschritt in einem nicht-gemischten bzw. zentrifugierten Zustand befinden soll, zu automatisieren. Die eingangs beschriebenen Fehlerquellen werden dadurch vermieden. Des Weiteren spart die Automatisierung eine signifikante Menge an Arbeitszeit ein.

Unter einem automatisierten Erkennen wird dabei insbesondere verstanden, dass für die Durchführung des Verfahrens kein manueller Eingriff mehr nötig ist. Insbesondere wird darunter verstanden, dass das Ergebnis des Verfahrens, also die Feststellung des Zustands der Probe, nicht von einer Beurteilung durch einen Menschen abhängt.

Bei der Probe handelt es sich insbesondere um eine Blutprobe. Es kann sich jedoch auch um andere Proben handeln, insbesondere aus dem medizinischen Bereich.

Bei dem nicht-gemischten Zustand handelt es sich bevorzugt um einen Zustand, in welchem die Probe in einzelne unterschiedliche Phasen aufgetrennt ist. Ein solcher nicht-gemischter Zustand kann insbesondere dadurch erreicht werden, dass die Probe mittels einer Zentrifuge bearbeitet wurde, denn beim Zentrifugieren wird eine Probe typischerweise in einzelne Phasen unterschiedlicher Dichte aufgetrennt. Im Beispiel einer Blutprobe trennt sich typischerweise das vorher vorhandene Vollblut in einen Blutkuchen mit den zellulären Bestandteilen des Bluts am unteren Ende des Probenröhrchens einerseits und flüssiges Blutplasma über dem Blutkuchen andererseits auf.

In einem gemischten Zustand ist insbesondere eine Phase des Phasentyps Vollblut nicht aufgetrennt. Der Feststellung eines gemischten Zustands steht es nicht entgegen, wenn sich zusätzlich zu einer Phase des Phasentyps Vollblut noch eine Phase des Phasentyps Gel in dem Probenröhrchen befindet. Das Gel wird nämlich typischerweise - wie weiter oben bereits erwähnt wurde - schon bei der Herstellung des Probenröhrchens in das Probenröhrchen verbracht und befindet sich somit am unteren Ende des Probenröhrchens. Wird nun Blut in das Probenröhrchen gefüllt, so vermischt sich dieses typischerweise nicht mit dem Gel. Vielmehr verbleibt das Blut zunächst als Vollblut über dem Gel.

Es sei auch angemerkt, dass Vollblut aufgrund der natürlichen Blutsenkung im Lauf der Zeit eine gewisse Auftrennung von sich aus durchführt, wobei am oberen Ende einer solchen Phase im Wesentlichen Blutplasma verbleibt und sich unterhalb des Blutplasmas eine Phase ausbildet, in welcher die zellulären Bestandteile des Bluts verdichtet sind. Die letztgenannte Phase kann auch als Pseudo-Blutkuchen bezeichnet werden und ist von einem wirklichen Blutkuchen unter Umständen nicht zu unterscheiden. Es wird weiter unten näher darauf eingegangen werden, wie diesem Phänomen in einer Weiterbildung des Verfahrens Rechnung getragen werden kann.

Beim Schritt des Ermittelns zumindest einer Eigenschaft an einer jeweiligen Position kann mindestens eine von mehreren Eigenschaften ermittelt werden, welche einen Rückschluss auf den jeweiligen Phasentyp zulassen. Hierzu werden an der jeweiligen Position eine erste Transmission (bzw. ein Transmissionsgrad oder ein Transmissionsfaktor oder -koeffizient) der Probe quer zur Längsrichtung bei einer ersten Wellenlänge als erste Eigenschaft und an der jeweiligen Position eine zweite Transmission (bzw. ein Transmissionsgrad oder ein Transmissionsfaktor oder -koeffizient) der Probe quer zur Längsrichtung bei einer zweiten Wellenlänge als zweite Eigenschaft ermittelt. Unter einer Transmission wird hierbei eine Größe für die Durchlässigkeit eines Mediums für Wellen, insbesondere elektromagnetische Wellen, verstanden. Es kann sich dabei um den Anteil von elektromagnetischer Strahlung (Licht) handeln, welcher durch die Probe und das umgebende Probenröhrchen, gegebenenfalls auch durch ein Etikett, hindurchtritt. Zur Ermittlung der Transmission kann das Probenröhrchen mit der darin befindlichen Probe beispielsweise mit Licht einer bestimmten Wellenlänge bestrahlt werden, wobei ein bezüglich des Probenröhrchens zur Lichtquelle gegenüberliegender Detektor misst, welcher Anteil des ausgestrahlten Lichts durch die Probe und das Probenröhrchen hindurchgegangen ist.

Insbesondere eignet sich für den vorgesehenen Zweck bei einer Blutprobe für die Ermittlung der ersten Transmission die Verwendung von Licht mit einer Wellenlänge zwischen 400 nm und 1200 nm und für die zweite Transmission die Verwendung von Licht mit einer Wellenlänge zwischen 1300 nm und 1700 nm. Hierbei handelt es sich um Werte, welche eine Unterscheidung der in Frage kommenden Phasentypen besonders zuverlässig zulassen. Dies liegt daran, dass Blutplasma, welches überwiegend aus Wasser besteht, bei Verwendung der genannten Wellenlängen eine hohe erste Transmission und eine niedrige zweite Transmission aufweist. Hierbei macht man sich die Eigenschaft der hohen Absorption von Wasser im infraroten Spektralbereich bei gleichzeitig niedriger Absorption im sichtbaren Spektralbereich zunutze, welche bei keinem anderen typischerweise in Frage kommenden Phasentyp vorkommt.

Bei einer Phase des Phasentyps Blutkuchen wird bei beiden Wellenlängen nahezu keine Strahlung durchgelassen, so dass die Transmission extrem niedrig ist. Bei einer Phase des Phasentyps Gel wird bei beiden Wellenlängen ein relativ hoher Teil der Strahlung durchgelassen, so dass die Transmission vergleichsweise hoch, jedoch bei der ersten Wellenlänge niedriger als bei Plasma ist. Bei einer Phase des Phasentyps Vollblut wird bei beiden Wellenlängen nur ein geringer Teil der Strahlung durchgelassen, so dass die Transmission vergleichsweise niedrig ist, jedoch höher als bei einer Phase des Phasentyps Blutkuchen. Die beschriebenen Eigenschaften ermöglichen eine zuverlässige Unterscheidung der unterschiedlichen Phasentypen.

Eine Transmission kann beispielsweise in Prozent angegeben werden. So bedeutet beispielsweise ein Wert von 80 %, dass ein Anteil von 80 % des auf einer Seite des Probenröhrchens eingestrahlten Lichts durch das Probenröhrchen und die Probe hindurchtritt, wohingegen 20 % dieses Lichts absorbiert oder reflektiert werden.

Es können bereits vorhandene Transmissionsmessmittel, die beispielsweise herkömmlich zur Grenzflächenbestimmung dienen, auch für die Zustandserkennung bzw. Zentrifugierungserkennung verwendet werden, ohne dass hierzu weiterer apparativer Aufwand notwendig ist.

Nachfolgend werden einige bevorzugte Ausführungsformen des erfindungsgemäßen Verfahrens beschrieben, mit welchen in bevorzugter Weise bestimmte Phasentypen ermittelt werden können. Es sei verstanden, dass die beschriebenen Ausführungsformen beliebig untereinander kombiniert werden können, so dass entsprechend mehr unterschiedliche Phasentypen erkannt werden können.

Gemäß einer Ausführung wird, wenn die erste Transmission oberhalb eines ersten Plasmaschwellenwerts liegt und die zweite Transmission unterhalb eines zweiten Plasmaschwellenwerts liegt, der Phasentyp Plasma (oder Serum) ermittelt. Dies trägt der bereits weiter oben beschriebenen Tatsache Rechnung, dass die Wellenlänge des für die Ermittlung der ersten Transmission verwendeten Lichts bevorzugt in einem Spektralbereich liegt, bei welchem das Plasma relativ gut transmittiert, wohingegen die Wellenlänge des für die Ermittlung der zweiten Transmission verwendeten Lichts bevorzugt in einem Spektralbereich liegt, in welchem das Plasma verhältnismäßig schlecht transmittiert. Der erste Plasmaschwellenwert kann somit einen verhältnismäßig hohen zweistelligen Prozentwert aufweisen, während der zweite Plasmaschwellenwert einen relativ niedrigen Wert aufweisen kann. Durch die Kombination einer hohen ersten Transmission und einer niedrigen zweiten Transmission kann der Phasentyp Plasma zuverlässig ermittelt werden, da eine solche Kombination bei keinem anderen typischen Phasentyp vorkommt.

Es sei verstanden, dass nicht zwangsläufig die erste Transmission und die zweite Transmission separat ausgewertet werden müssen. Vielmehr ist es auch möglich, einen Quotienten der zweiten Transmission und der ersten Transmission zu bilden, wobei in diesem Fall auch davon gesprochen werden kann, dass die erste Transmission eine Referenz für die zweite Transmission bildet. Es versteht sich, dass als Rechengröße der Quotient oder ein Kehrwert des Quotienten verwendet werden kann. Der Quotient ist vergleichsweise unabhängig von einer Schichtdicke eines Probenröhrchenmaterials, welches üblicherweise aus transparentem Kunststoff ausgebildet ist, und einer Anzahl von Labeln oder Etiketten, die auf dem Probenröhrchen aufgeklebt sind. Damit erlaubt der Quotient eine vorteilhafte Bestimmung der jeweiligen Phasentypen.

Gemäß einer weiteren Ausführung wird, wenn die erste Transmission oberhalb eines unteren ersten Gelschwellenwerts und unterhalb eines oberen ersten Gelschwellenwerts liegt und die zweite Transmission oberhalb eines unteren zweiten Gelschwellenwerts und unterhalb eines oberen zweiten Gelschwellenwerts liegt, der Phasentyp Gel ermittelt. Dies trägt der weiter oben bereits beschriebenen Tatsache Rechnung, dass Gel die beiden für die Ermittlung der ersten und der zweiten Transmission verwendeten Wellenlängen nur sehr wenig absorbiert. Durch die jeweiligen unteren Gelschwellenwerte werden Abgrenzungen zu schwächer transmittierenden Phasentypen wie Blutkuchen oder Vollblut vorgenommen. Durch die jeweiligen oberen Gelschwellenwerte werden Abgrenzungen zu noch stärker transmittierenden Phasen wie Luft vorgenommen. Die erwähnten Gelschwellenwerte können in einem verhältnismäßig hohen zweistelligen Prozentbereich liegen, wobei jeweils der obere Gelschwellenwert größer ist als der untere Gelschwellenwert.

Gemäß einer Ausführung wird, wenn die erste Transmission unterhalb eines ersten Blutkuchenschwellenwerts liegt und die zweite Transmission unterhalb eines zweiten Blutkuchenschwellenwerts liegt, der Phasentyp Blutkuchen ermittelt. Dies trägt der weiter oben bereits beschriebenen Tatsache Rechnung, dass Blutkuchen derjenige Phasentyp ist, welcher am schlechtesten transmittiert. Deshalb werden typischerweise die beiden Blutkuchenschwellenwerte die jeweils niedrigsten vorkommenden Schwellenwerte sein. Bei einer äußerst geringen Transmission oder auch bei einer vollständigen Blockierung des Lichts beider Wellenlängen kann zuverlässig auf den Phasentyp Blutkuchen geschlossen werden.

Gemäß einer Ausführung wird, wenn die erste Transmission oberhalb eines unteren ersten Vollblutschwellenwerts und unterhalb eines oberen ersten Vollblutschwellenwerts liegt und die zweite Transmission oberhalb eines unteren zweiten Vollblutschwellenwerts und unterhalb eines oberen zweiten Vollblutschwellenwerts liegt, der Phasentyp Vollblut ermittelt. Damit wird der weiter oben bereits erwähnten Tatsache Rechnung getragen, dass Vollblut zwar etwas besser transmittiert als Blutkuchen, jedoch schlechter als Gel. Die erwähnten Vollblutschwellenwerte werden somit typischerweise zwischen einem jeweiligen Blutkuchenschwellenwert und einem jeweiligen unteren Gelschwellenwert liegen.

Nachfolgend werden einige mögliche Ausführungen des Verfahrens beschrieben, wobei sich diese darauf beziehen, wie aus erkannten Phasen bzw. Phasentypen an unterschiedlichen Positionen auf einen gemischten Zustand oder auf einen nicht-gemischten Zustand geschlossen werden kann. Es sei verstanden, dass die erwähnten Ausführungen auch beliebig miteinander kombiniert werden können, so dass anhand möglichst vieler Charakteristika auf den Zustand der Probe geschlossen werden kann. Dadurch kann die Zuverlässigkeit erhöht werden. Beispielsweise kann bei abweichenden Ergebnissen eine Fehlermeldung ausgegeben werden oder es kann vorsorglich ein gemischter Zustand ermittelt werden, wenn auch nur eine der möglichen Ausführungen einen gemischten Zustand ermittelt.

Gemäß einer Ausführung wird bei Ermittlung eines Phasentyps Gel an einer Position zwischen einem geschlossenen bzw. unteren Ende des Probenröhrchens und einer oberen Gelposition ein gemischter Zustand ermittelt.

Die obere Gelposition ist bevorzugt diejenige Position, bis zu welcher sich Gel im Auslieferungszustand des Probenröhrchens typischerweise vom geschlossenen bzw. unteren Ende des Probenröhrchens aus nach oben erstreckt. Es ist anders ausgedrückt die vom Hersteller vorgegebene Füllhöhe. Sie kann beispielsweise in einer Längeneinheit ausgehend vom geschlossenen bzw. unteren Ende des Probenröhrchens angegeben werden.

Wie bereits weiter oben erwähnt befindet sich Gel in einem Probenröhrchen vor dem Zentrifugieren typischerweise im untersten Bereich des Probenröhrchens, da es bereits vom Hersteller des Probenröhrchens in das Probenröhrchen eingefüllt wurde und auch beim nachträglichen Einfüllen von Blut in die Probe zunächst an dieser Position verbleibt. Erst wenn die Probe in einen nicht-gemischten Zustand überführt wird, beispielsweise durch Zentrifugieren, verschiebt sich das Gel dichtebedingt in eine Position, welche vom geschlossenen bzw. unteren Ende des Probenröhrchens um einen messbaren Abstand entfernt ist. Zwischen dem Gel und dem geschlossenen bzw. unteren Ende des Probenröhrchens wird bei einer nicht-gemischten, also beispielsweise zentrifugierten, Probe typischerweise der Blutkuchen angeordnet sein, welcher das dichteste vorhandene Material ist. Aus dem Vorhandensein von Gel am untersten Ende des Probenröhrchens kann somit auf einen unzentrifugierten Zustand geschlossen werden.

Gemäß einer Ausführung wird bei Ermittlung eines Phasentyps Vollblut ein gemischter Zustand ermittelt. Dies trägt der Tatsache Rechnung, dass in einer Probe, welche einer ordnungsgemäßen Auftrennung in die einzelnen Phasen unterzogen wurde, beispielsweise durch Zentrifugieren, der Phasentyp Vollblut typischerweise nicht mehr vorhanden ist. Vielmehr sind die Bestandteile des Vollbluts, also insbesondere Plasma einerseits und zelluläre Bestandteile andererseits, in Phasen der Phasentypen Plasma und Blutkuchen getrennt. Aus dem Vorhandensein des Phasentyps Vollblut kann somit zuverlässig auf einen gemischten Zustand, also beispielsweise eine nicht zentrifugierte Probe, geschlossen werden.

Gemäß einer Ausführung wird bei Ermittlung zumindest dreier fester oder flüssiger Phasen unterschiedlicher Typen ein nicht-gemischter Zustand ermittelt. Dies basiert darauf, dass eine Probe im gemischten Zustand, also beispielsweise vor dem Zentrifugieren, im Normalfall lediglich zwei Phasen aufweisen kann. Sofern es sich um ein Probenröhrchen ohne eingefülltes Gel handelt, weist eine unzentrifugierte Probe lediglich eine Phase auf, nämlich Vollblut. Sofern es sich bei dem Probenröhrchen um ein Probenröhrchen handelt, in welches Gel eingefüllt wurde, weist die Probe typischerweise zwei Phasen auf, nämlich Vollblut und darunterliegend Gel. Nur durch Auftrennen der Phase des Phasentyps Vollblut in Phasen der Phasentypen Plasma und Blutkuchen kann eine dritte Phase erzeugt werden.

Gemäß einer Ausführung wird bei Ermittlung eines Phasentyps Blutkuchen eine Länge der Phase des Phasentyps Blutkuchen entlang der Längsrichtung ausgehend von einem geschlossenen bzw. unteren Ende des Probenröhrchens und ferner eine Länge aller festen oder flüssigen Phasen im Probenröhrchen ausgehend von dem geschlossenen bzw. unteren Ende des Probenröhrchens ermittelt, wobei anschließend ein Verhältnis der Länge des Blutkuchens zur Länge aller festen oder flüssigen Phasen im Probenröhrchen berechnet wird. Ein nicht-gemischter Zustand wird ermittelt, wenn das Verhältnis unterhalb eines Trennungsschwellenwerts liegt. Ein gemischter Zustand wird ermittelt, wenn das Verhältnis oberhalb des Trennungsschwellenwerts liegt.

Von der Länge aller festen oder flüssigen Phasen kann dabei gemäß einer alternativen Ausführung die Länge einer Phase des Phasentyps Gel abgezogen werden.

Bei der Länge der Phase des Phasentyps Blutkuchen wird typischerweise eine Länge einer Phase des Phasentyps Gel, welche sich ebenfalls in der Probe befinden kann, nicht mitgerechnet.

Durch die eben beschriebene Ausführung wird der bereits weiter oben beschriebenen Tatsache Rechnung getragen, dass sich eingefülltes Vollblut auch ohne Einwirkung, also insbesondere auch ohne Zentrifugieren, im Laufe der Zeit in zwei unterschiedliche wahrnehmbare Phase auftrennt. Dies liegt an der bekannten Blutsenkung, welche im normalen Fall bei etwa 20 bis 40 mm pro Stunde liegt. Dies entspricht der Geschwindigkeit, mit welcher sich eine als Plasma wahrnehmbare Phase oberhalb einer Phase mit im Vergleich zum ursprünglichen Vollblut erhöhter Konzentration zellulärer Bestandteile ausbildet. Diese Phase kann in ihrer Transmissionscharakteristik ähnlich oder identisch zu einem Blutkuchen sein, so dass sie nicht zuverlässig von einem tatsächlichen Blutkuchen, welcher durch Zentrifugieren entstanden ist, unterschieden werden kann.

Durch die beschriebene Vorgehensweise wird die Sicherheit in dem Fall erhöht, dass die Probe bereits eine längere Zeit gelagert wurde, bevor das erfindungsgemäße Verfahren angewendet wird. Dies ist insbesondere dann besonders vorteilhaft, wenn sich kein Gel im Probenröhrchen befindet, da in diesem Fall die Unterscheidung durch die Position von Gel und Blutkuchen relativ zueinander nicht möglich ist.

Der Trennungsschwellenwert liegt typischerweise im niedrigen zweistelligen Prozentbereich. Nur wenn die ermittelte Phase des Phasentyps Blutkuchen einen verhältnismäßig kleinen Teil der Gesamterstreckung aller festen oder flüssigen Phasen einnimmt, kann mit hinreichender Sicherheit davon ausgegangen werden, dass die Probe tatsächlich absichtlich aufgetrennt, also beispielsweise zentrifugiert wurde. Sofern die ermittelte Phase des Phasentyps Blutkuchen einen überwiegenden Teil der Gesamterstreckung aller festen und flüssigen Phasen in dem Probenröhrchen einnimmt, kann eher davon ausgegangen werden, dass das Probenröhrchen lediglich eine gewisse Zeit gelagert wurde und der ermittelte vermeintliche Blutkuchen in Wirklichkeit eine verdichtete Phase Vollblut ist. In diesem Fall müsste das Probenröhrchen zunächst zentrifugiert werden, bevor weitere Analyseschritte durchgeführt werden können.

Es sei darauf hingewiesen, dass die eben beschriebene Ausführung des Verfahrens, welche eine gewisse Sicherheit gegen die falsche Zustandsermittlung bei lange gelagerten Proben bietet, besonders vorteilhaft mit der weiter oben beschriebenen Ausführung kombiniert werden kann, welche auf der Ermittlung von drei unterschiedlichen Phasentypen basiert. Sofern beispielsweise drei unterschiedliche Phasentypen erkannt werden, wovon eine Phase den Phasentyp Blutkuchen aufweist, kann durch die eben beschriebene Vorgehensweise verhindert werden, dass eine Probe als nicht-gemischt klassifiziert wird, obwohl sie in Wirklichkeit lediglich eine längere Zeit gelagert wurde. Die Ermittlung anhand des Trennungsschwellenwerts kann somit das Ergebnis der Ermittlung anhand des Vorhandenseins dreier Phasen überstimmen.

Gemäß einer Ausführung wird bei Ermittlung eines Phasentyps Gel, wenn die Phase Gel um einen Mindestabstand von einem geschlossenen Ende des Probenröhrchens beabstandet ist, ein nicht-gemischter Zustand ermittelt. Dies trägt der weiter oben bereits beschriebenen Tatsache Rechnung, dass sich Gel, sofern es sich überhaupt in dem Probenröhrchen befindet, bei einem unzentrifugierten Probenröhrchen weiterhin ganz unten in dem Probenröhrchen befindet, also die tiefstliegendste Phase ausmacht. Erst durch aktives Trennen, beispielsweise durch Zentrifugieren, bildet sich eine Phase Blutkuchen unterhalb des Gels aus, wobei das Gel von dem geschlossenen Ende des Probenröhrchens entfernt wird. Es verbleibt in einem Zustand weiter oberhalb des Blutkuchens. Aus dem Vorhandensein von Gel mit einem Mindestabstand, welcher typischerweise der typischen Erstreckung eines Blutkuchens entspricht, kann somit zuverlässig auf einen nicht-gemischten Zustand geschlossen werden.

Bevorzugt wird die zumindest eine Eigenschaft der Probe entlang der Längsrichtung kontinuierlich ermittelt. Dies bedeutet insbesondere, dass eine Vielzahl von Messwerten entlang der Längsrichtung für die Eigenschaft aufgenommen wird, so dass die Messpunkte in Längsrichtung um einen Abstand voneinander beabstandet sind, welcher erheblich kleiner ist als typische Größenordnungen, in welchen sich die Eigenschaft beispielsweise durch einen Übergang zwischen zwei Phasen ändert. Sofern eine zweite Eigenschaft ermittelt wird, gilt für diese bevorzugt das gleiche wie für die erste Eigenschaft. Damit kann eine besonders zuverlässige Ermittlung des Zustands der Probe erreicht werden.

Die Erfindung betrifft des Weiteren eine Vorrichtung zum Analysieren von Proben, insbesondere Blutproben, welche in Probenröhrchen enthalten sind. Die Vorrichtung weist Folgendes auf:
- eine Vorrichtung zum Ermitteln zumindest einer Eigenschaft der Probe an einer Mehrzahl von unterschiedlichen Positionen entlang einer Längsrichtung des Probenröhrchens, und
- eine elektronische Steuerungsvorrichtung, welche Prozessormittel und diesen zugeordnete Speichermittel aufweist, wobei in den Speichermitteln Instruktionen gespeichert sind, bei deren Ausführung durch die Prozessormittel die Vorrichtung ein erfindungsgemäßes Verfahren ausführt.

Bei dem erfindungsgemäßen Verfahren, welches durch die Vorrichtung ausgeführt wird, kann auf alle mit Bezug auf das erfindungsgemäße Verfahren beschriebenen Varianten zurückgegriffen werden. Erläuterte Vorteile gelten entsprechend.

Die Vorrichtung kann zumindest eine erste Lichtquelle und eine zweite Lichtquelle aufweisen, wobei die erste Lichtquelle im Betrieb Licht mit einer ersten Wellenlänge zwischen 400 nm und 1200 nm emittiert und die zweite Lichtquelle im Betrieb Licht mit einer zweiten Wellenlänge zwischen 1300 nm und 1700 nm emittiert. Die Vorrichtung kann weiter einen oder mehrere Detektoren zur Detektion des von der ersten Lichtquelle emittierten Lichts und zur Detektion des von der zweiten Lichtquelle emittierten Lichts jeweils nach Durchgang durch das Probenröhrchen quer zur Längsrichtung an einer Position entlang der Längsrichtung aufweisen. Die erste und die zweite Lichtquelle können in Längsrichtung des Probenröhrchens verfahrbar angeordnet sein.

Mittels der beschriebenen Lichtquellen und Detektoren können die erste Transmission und die zweite Transmission, wie weiter oben beschrieben, ermittelt werden. Insbesondere kann die Transmission des Lichts mit einer Wellenlänge zwischen 400 nm und 1200 nm die erste Transmission sein und die Transmission des Lichts mit einer Wellenlänge zwischen 1300 nm und 1700 nm kann die zweite Transmission sein.

Zur Detektion der Transmissionen kann entweder ein Detektor verwendet werden, welcher für beide relevanten Spektralbereiche das transmittierte Licht detektieren kann, oder es können zwei separate Detektoren verwendet werden.

Die Lichtquellen können entweder relativ zu einem sich in Ruhe befindlichen Probenröhrchen verfahren werden oder das Probenröhrchen kann relativ zu sich in Ruhe befindlichen Lichtquellen verfahren werden. Auch ein gleichzeitiges Verfahren ist möglich.

Die Erfindung betrifft des Weiteren ein Laborautomatisierungssystem, welches Folgendes aufweist:
- eine Aufnahmestation für Probenröhrchen,
- eine Zentrifuge,
- Mittel zum Verbringen des Probenröhrchens aus der Aufnahmestation in die Zentrifuge,
- eine erfindungsgemäße Vorrichtung zum Analysieren von Proben und
- Mittel zum Verbringen von Probenröhrchen aus der Zentrifuge in die Vorrichtung zum Analysieren von Proben,
wobei die Steuerungsvorrichtung der Vorrichtung zum Analysieren von Proben ausgebildet ist, das erfindungsgemäße Verfahren vor weiteren Analyseschritten durchzuführen und für den Fall, dass dabei ein gemischter Zustand ermittelt wird, keine weiteren Analyseschritte durchzuführen und bevorzugt eine Fehlermeldung auszugeben.

Durch das erfindungsgemäße Laborautomatisierungssystem kann das erfindungsgemäße Verfahren in vorteilhafter Weise für ein weitgehend automatisiertes System angewendet werden.

Bei der Aufnahmestation für Probenröhrchen kann es sich beispielsweise um einen üblichen Probenröhrchenhalter handeln. Bei der Zentrifuge kann es sich beispielsweise um eine übliche Laborzentrifuge handeln. Bei den Mitteln zum Verbringen von Probenröhrchen aus der Aufnahmestation in die Zentrifuge kann es sich beispielsweise um einen Roboter handeln, welcher Probenröhrchen aus der Aufnahmestation entnimmt und in die Zentrifuge einsetzt. Bei der Vorrichtung zum Analysieren von Proben kann auf alle beschriebenen Ausführungen zurückgegriffen werden, wobei erläuterte Vorteile entsprechend gelten. Insbesondere kann es sich bei der Vorrichtung zum Analysieren von Proben um die weiter oben beschriebene Ausführung mit zwei Lichtquellen unterschiedlicher Wellenlänge handeln. Bei den Mitteln zum Verbringen von Probenröhrchen aus der Zentrifuge in die Vorrichtung zum Analysieren von Proben kann es sich beispielsweise um einen Roboter handeln, welcher Probenröhrchen aus der Zentrifuge entnimmt und in die Vorrichtung zum Analysieren von Proben einsetzt. Hierzu kann die Vorrichtung zum Analysieren von Proben beispielsweise eine ähnliche oder identische Aufnahmestation wie weiter oben beschrieben aufweisen. Beispielsweise können die Mittel zum Verbringen von Probenröhrchen aus der Zentrifuge in die Vorrichtung zum Analysieren von Proben und die Mittel zum Verbringen von Probenröhrchen aus der Aufnahmestation in die Zentrifuge identisch sein. Beispielsweise kann es sich hierbei um den gleichen Roboter handeln.

Bei dem von dem erfindungsgemäßen Laborautomatisierungssystem anzuwendenden erfindungsgemäßen Verfahren kann auf alle oben beschriebenen Ausführungen zurückgegriffen werden. Erläuterte Vorteile gelten entsprechend.

Das erfindungsgemäße Laborautomatisierungssystem ermöglicht eine vollautomatisierte Analyse von Proben, wobei das Laborautomatisierungssystem auch den Schritt des Zentrifugierens automatisch ausführt. Gleichzeitig ist eine Sicherheit vorgesehen, welche verhindert, dass nicht zentrifugierte Proben analysiert werden, wobei solche beispielsweise bei einer Fehlfunktion eines der Mittel zum Verbringen von Proben, beispielsweise eines Roboters, auftreten können oder durch menschliche Eingriffe ausgelöst werden können. Die Sicherheit des Gesamtsystems wird auf diese Weise erheblich erhöht.

Durch eine ausgegebene Fehlermeldung kann ein Benutzer des Laborautomatisierungssystems auf die Fehlfunktion hingewiesen werden, wobei er in diesem Fall manuell eingreifen kann.

Die Vorrichtung zum Analysieren von Proben kann eine Analyseeinrichtung aufweisen, welche gewünschte zu analysierende Messwerte, beispielsweise einen Blutalkoholgehalt, ermittelt. Hierzu kann die Vorrichtung beispielsweise Mittel zum Entnehmen von Flüssigkeiten oder Festkörpern aus dem Inneren des Probenröhrchens sowie bekannte Analysegeräte, beispielsweise spektroskopische Analysegeräte, aufweisen.

### Kurze Beschreibung der Zeichnung

Die Erfindung wird nachfolgend unter Bezugnahme auf die Zeichnungen detailliert beschrieben. Dabei zeigen schematisch:
- Fig. 1a bis 1d:: Probenröhrchen mit unterschiedlichen Proben,
- Fig. 2:: eine Vorrichtung zum Analysieren von Proben,
- Fig. 3a und 3b:: schematische Darstellungen typischer Schwellenwerte und
- Fig. 4:: ein Laborautomatisierungssystem.

### Detaillierte Beschreibung der Ausführungsbeispiele

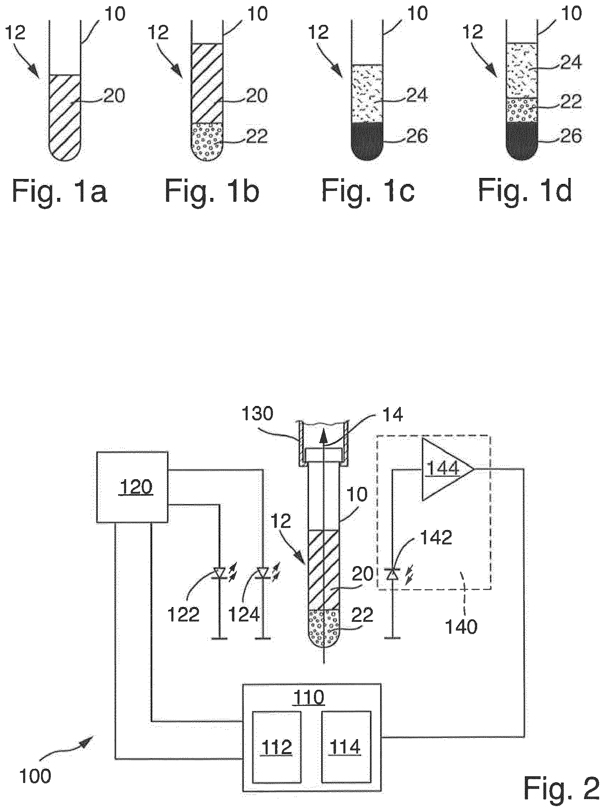

Die Fig. 1a bis 1d zeigen Probenröhrchen 10 mit unterschiedlichen darin enthaltenen Proben 12. Die Proben 12 weisen jeweils eine oder mehrere Phasen auf, wobei im Fall von mehreren Phasen die jeweiligen Phasen unterschiedliche Phasentypen haben. Nachfolgend wird in der Regel auf die explizite Bezeichnung als "Phase" oder "Phasentyp" verzichtet, um die Lesbarkeit zu verbessern. Vielmehr wird nachfolgend meistens lediglich der jeweilige Phasentyp angegeben.

Fig. 1 a zeigt ein Probenröhrchen 10 mit einer Probe 12, welche vollständig aus Vollblut 20 besteht. Das Vollblut 20 befindet sich in einem gemischten Zustand. Dies weist darauf hin, dass die Probe 12 in dem Probenröhrchen 10 noch nicht aufgetrennt wurde, also beispielsweise noch nicht zentrifugiert wurde.

Fig. 1b zeigt ein Probenröhrchen 10 mit einer Probe 12, welche sowohl ein Gel 22 wie auch Vollblut 20 aufweist. Vollblut 20 ist oberhalb des Gels 22 angeordnet. Wie bereits weiter oben erwähnt wird das Gel 22 bei der Herstellung des Probenröhrchens 10 bereits vom Hersteller in das Probenröhrchen 10 gefüllt und verbleibt auch bei Einfüllen von Blut in dem Probenröhrchen 10 an dieser Stelle. Eine wesentliche Vermischung zwischen dem Vollblut 20 und dem Gel 22 findet nicht statt. Somit handelt es sich bei der Probe 12 von Fig. 1b ebenfalls um eine gemischte, also noch unzentrifugierte Probe, wobei im Unterschied zur Probe 12 von Fig. 1 a zusätzlich Gel 22 vorhanden ist.

Fig. 1c zeigt ein Probenröhrchen 10 mit einer Probe 12, welche der bereits aus Fig. 1 a bekannten Probe 12 entspricht, nun jedoch in einem nicht-gemischten, also zentrifugierten Zustand ist. Anders ausgedrückt wurde das Probenröhrchen 10 von Fig. 1a zentrifugiert, um den Zustand von Fig. 1c zu erhalten.

Wie in Fig. 1c zu erkennen ist, befindet sich am unteren Ende des Probenröhrchens 10 ein Blutkuchen 26. Dieser weist eine besonders hohe Dichte auf. Oberhalb des Blutkuchens 26 befindet sich ein Plasma 24, welches im Wesentlichen aus den wässrigen, nicht zellulären Bestandteilen des Vollbluts 20 besteht. Das Vollblut 20 wurde also in das Plasma 24 und den Blutkuchen 26 aufgetrennt.

Fig. 1d zeigt ein Probenröhrchen 10 mit einer Probe 12, welche insgesamt drei Phasen aufweist. Am unteren Ende befindet sich ein Blutkuchen 26. Unmittelbar darüber befindet sich ein Gel 22. Nochmals darüber befindet sich ein Plasma 24.

Die Probe 12 von Fig. 1d entspricht der bereits aus Fig. 1b bekannten Probe 12 in einem nicht-gemischten, also zentrifugierten Zustand. Anders ausgedrückt wurde das Probenröhrchen 10 von Fig. 1b zentrifugiert, um den in Fig. 1d dargestellten Zustand zu erhalten. Wie auch beim Übergang vom in Fig. 1a dargestellten Zustand zu dem in Fig. 1c dargestellten Zustand wurde auch beim Übergang vom in Fig. 1b dargestellten Zustand zum in Fig. 1d dargestellten Zustand beim Zentrifugieren das Vollblut 20 in den Blutkuchen 26 und das Plasma 24 aufgeteilt. Der Blutkuchen 26 nimmt auch hier die zellulären Bestandteile auf, wohingegen das Plasma 24 die wässrigen, nicht zellulären Bestandteile aufnimmt. Das Gel 22 ordnet sich aufgrund seiner Dichte zwischen dem Blutkuchen 26 und dem Plasma 24 an.

Es sei erwähnt, dass bei der obigen Beschreibung der Fig. 1a bis 1d jeweils eine Phase des Phasentyps Luft nicht näher betrachtet wurde, welche sich jeweils oberhalb der wässrigen und flüssigen Phasen in dem Probenröhrchen 10 befindet. Der Phasentyp Luft spielt für die Durchführung des erfindungsgemäßen Verfahrens im Normalfall keine Rolle, da das Vorhandensein von Luft nicht auf einen gemischten oder nicht-gemischten Zustand hinweist.

Fig. 2 zeigt eine Vorrichtung 100 zum Analysieren von Proben. In der Vorrichtung 100 befindet sich ein Probenröhrchen 10 mit einer darin enthaltenen Probe 12. Wie in Fig. 2 ebenfalls zu erkennen ist, weist das Probenröhrchen 10 eine Längsrichtung 14 auf, wobei das Probenröhrchen 10 über seine Erstreckung entlang der Längsrichtung 14 radialsymmetrisch bezüglich der Längsrichtung 14 ausgebildet ist.

Die Vorrichtung 100 weist einen Mikrocontroller 110 mit einer Lichtquellenansteuereinheit 112 und einer Recheneinheit 114 auf. Die Lichtquellenansteuereinheit 112 ist mit einem Treiber 120 verbunden, welcher wiederum mit einer ersten Leuchtdiode 122 und einer zweiten Leuchtdiode 124 verbunden ist. Die Lichtquellenansteuereinheit 112 kann an den Treiber 120 Signale ausgeben, woraufhin der Treiber 122 die beiden Leuchtdioden 122, 124 wie von der Lichtquellenansteuereinheit 112 gewünscht ein- und ausschaltet.

Die erste Leuchtdiode 122 emittiert im Betrieb Licht mit einer ersten Wellenlänge von 980 nm. Alternativ könnten beispielsweise auch 940 nm verwendet werden. Die zweite Leuchtdiode 124 emittiert im Betrieb Licht mit einer zweiten Wellenlänge von 1550 nm. Beide Leuchtdioden 122, 124 sind unmittelbar neben dem Probenröhrchen 10 angeordnet und strahlen ihr Licht derart senkrecht zur Längsrichtung 14 seitlich in das Probenröhrchen 10 hinein, dass das Licht in etwa durch den Mittelpunkt des kreisförmigen Probenröhrchenquerschnitts verläuft.

Bezüglich des Probenröhrchens 10 gegenüberliegend zu den Leuchtdioden 122, 124 ist ein Lichtempfänger 140 angeordnet, welcher einen Sensor 142 sowie einen Operationsverstärker 144 aufweist. Bei dem Sensor 142 handelt es sich vorliegend um einen InGaAs-Sensor, welcher die vorteilhafte Eigenschaft aufweist, dass er Licht mit einer Wellenlänge ab etwa 850 nm detektieren kann. Der Operationsverstärker 144 bereitet die von dem Sensor 142 erhaltenen Signale auf und leitet sie weiter an den Mikrocontroller 110. Dies ermöglicht eine Messung einer Intensität des aus dem Probenröhrchen 10 austretenden Lichts und somit die Bestimmung einer Transmission bzw. eines Transmissionsverhältnisses oder Intensitätsverhältnisses zwischen der ersten und der zweiten Wellenlänge.

Das Probenröhrchen 10 ist in einem Probenhalter 130 aufgenommen, welcher entlang der Längsrichtung 14 des Probenröhrchens 10, d.h. sowohl nach oben wie auch nach unten, verfahrbar ist. Damit kann die Transmission von durch zumindest eine der Leuchtdioden 122, 124 emittiertem Licht durch das Probenröhrchen 10 mit der darin befindlichen Probe 12 an einer Vielzahl von Positionen entlang der Längsrichtung 14, faktisch also kontinuierlich entlang der Längsrichtung 14, gemessen werden.

Die Recheneinheit 114 des Mikrocontrollers 110 ist dazu ausgebildet, ein erfindungsgemäßes Verfahren durchzuführen. Hierzu wird mittels des Probenhalters 130 das Probenröhrchen 10 entlang seiner Längsrichtung 14 von oben nach unten so durchgefahren, dass es über einen wesentlichen Teil seiner Erstreckung entlang der Längsrichtung 14 mittels der Leuchtdioden 122, 124 und dem Lichtempfänger 140 vermessen werden kann. Darunter wird vorliegend verstanden, dass an jeder Position abwechselnd die erste Leuchtdiode 122 und dann die zweite Leuchtdiode 124 eingeschaltet werden, wobei jeweils eine Lichtintensität bzw. eine Transmission, also ein Durchgang von Licht durch das Probenröhrchen 10 mit der darin befindlichen Probe 12, ermittelt wird. Es sei verstanden, dass die Reihenfolge des Einschaltens der beiden Leuchtdioden 122, 124 auch vertauscht werden kann. Ebenso kann das Probenröhrchen 10 auch von unten nach oben durchgefahren werden.

Jeweilige Messpunkte entlang der Längsrichtung 14 liegen dabei sehr nah beieinander, was insbesondere bedeutet, dass sie kleiner sind als eine typische Größenordnung, in welcher sich die Verhältnisse im Inneren des Probenröhrchens 10 ändern.

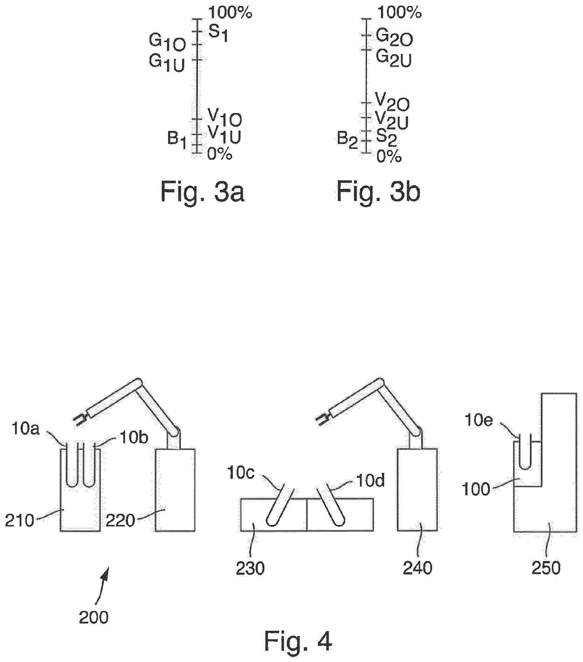

Durch das beschriebene Vorgehen erhält der Mikrocontroller 110 zwei Messkurven, welche an jeder Stelle entlang der Längsrichtung 14 des Probenröhrchens 10 die Transmission bei der ersten Wellenlänge und bei der zweiten Wellenlänge angeben. Nachdem dies durchgeführt wurde, kann der Mikrocontroller 110 bestimmen, ob die Probe 12 sich in einem nicht-gemischten oder in einem gemischten Zustand, also typischerweise in einem zentrifugierten oder einem unzentrifugierten Zustand befindet. Hierzu können die nachfolgend mit Bezug auf die Fig. 3a und 3b beschriebenen Schwellenwerte verwendet werden.

Die Fig. 3a und 3b zeigen schematisch typische Schwellenwerte für die Ermittlung jeweiliger Phasentypen. Dabei zeigt Fig. 3a die Schwellenwerte in Bezug auf die erste Wellenlänge, wohingegen Fig. 3b die Schwellenwerte in Bezug auf die zweite Wellenlänge zeigt.

Die beiden jeweils niedrigsten Schwellenwerte sind der erste Blutkuchenschwellenwert B₁ und der zweite Blutkuchenschwellenwert B₂. Sofern die Transmission jeweils unterhalb dieser Schwellenwerte liegt, kann von einem Blutkuchen ausgegangen werden.

In Fig. 3a befinden sich oberhalb des ersten Blutkuchenschwellenwerts B₁ ein unterer erster Vollblutschwellenwert V_{1U} und ein oberer erster Vollblutschwellenwert V_{1O}. Bei Fig. 3b befinden sich oberhalb des zweiten Blutkuchenschwellenwerts B₂ ein unterer zweiter Vollblutschwellenwert V_{2U} und ein oberer zweiter Vollblutschwellenwert V_{2O}, wobei jedoch im Gegensatz zu Fig. 3a zwischen dem zweiten Blutkuchenschwellenwert B₂ und dem unteren zweiten Vollblutschwellenwert V_{2U} ein zweiter Plasmaschwellenwert S₂ angeordnet ist.

Sofern sich die erste Transmission zwischen den beiden ersten Vollblutschwellenwerten V_{1U}, V_{1O} und die zweite Transmission zwischen den beiden zweiten Vollblutschwellenwerten V_{2U}, V_{2O} befindet, kann von einem Phasentyp Vollblut ausgegangen werden.

Jeweils oberhalb des jeweiligen oberen Vollblutschwellenwerts V_{1O}, V_{2O} befinden sich Gelschwellenwerte. Im Fall der Fig. 3a sind dies ein unterer erster Gelschwellenwert G_{1U} und ein oberer erster Gelschwellenwert G_{1O}. Im Fall der Fig. 3b sind dies ein unterer zweiter Gelschwellenwert G_{2U} und ein oberer zweiter Gelschwellenwert G_{2O}. Sofern sich die erste Transmission zwischen den beiden ersten Gelschwellenwerten G_{1U}, G_{1O} befindet und sich die zweite Transmission zwischen den beiden zweiten Gelschwellenwerten G_{2U}, G_{2O} befindet, kann von einem Phasentyp Gel ausgegangen werden.

In Fig. 3a ist weiter gezeigt, dass sich oberhalb des oberen ersten Gelschwellenwerts G_{1O} noch ein erster Plasmaschwellenwert S₁ befindet. Sofern die erste Transmission oberhalb des ersten Plasmaschwellenwerts S₁ liegt und die zweite Transmission unterhalb des zweiten Plasmaschwellenwerts S₂ liegt, kann von einem Phasentyp Plasma ausgegangen werden.

Nach Bestimmung der jeweiligen Phasen an den jeweiligen Positionen nimmt der Mikrocontroller 110 eine Bestimmung vor, ob die Probe 12 sich in einem gemischten oder einem nicht-gemischten Zustand befindet. Dabei wird folgendermaßen vorgegangen:
Sofern ein Phasentyp Blutkuchen 26 ermittelt wird, wird eine Länge des Blutkuchens 26 und auch eine Länge aller festen und flüssigen Phasen 20, 22, 24, 26 im Probenröhrchen 10 ermittelt. Anschließend wird das Verhältnis der Länge des Blutkuchens 20 zur Gesamtlänge aller festen und flüssigen Phasen 20, 22, 24, 26 ermittelt. Sofern dieses Verhältnis oberhalb eines Trennungsschwellenwerts liegt, wird ein gemischter Zustand ermittelt. Sofern das Verhältnis unterhalb des Trennungsschwellenwerts liegt, wird ein nicht-gemischter Zustand ermittelt. Damit wird dem bereits weiter oben beschriebenen Phänomen der natürlichen Blutsenkung Rechnung getragen und vermieden, dass eine lediglich aufgrund von Blutsenkung teilweise getrennte Probe als sich in einem nicht-gemischten Zustand befindlich klassifiziert wird.

Sofern ein Phasentyp Gel 22 ermittelt wird, wird weiter festgestellt, ob sich eine Phase des Phasentyps Gel 22 unmittelbar am unteren Ende, also benachbart zum geschlossenen Ende des Probenröhrchens 10, oder mit einem gewissen Mindestabstand von dem unteren Ende des Probenröhrchens 10 beabstandet befindet. Im ersteren Fall wird ein gemischter Zustand ermittelt. Im zweiten Fall wird ein nicht-gemischter Zustand ermittelt.

Sollten die beiden beschriebenen Ermittlungsverfahren unterschiedliche Ergebnisse liefern, wird eine Fehlermeldung ausgegeben. Sollten beide Ermittlungsverfahren einen nicht-gemischten Zustand ermitteln oder das erste Ermittlungsverfahren einen nicht-gemischten Zustand ermitteln und das zweite Ermittlungsverfahren mangels Vorhandenseins von Gel 22 nicht anwendbar sein, so wird bestimmt, dass die sich in dem Probenröhrchen 10 befindliche Probe 12 offenbar zentrifugiert wurde und analysiert werden kann. Sollten beide beschriebenen Ermittlungsverfahren einen gemischten Zustand ergeben oder das erste beschriebene Ermittlungsverfahren einen gemischten Zustand ergeben und das zweite Ermittlungsverfahren mangels Vorhandenseins von Gel 22 nicht anwendbar sein, so wird bestimmt, dass die sich in dem Probenröhrchen 10 befindliche Probe 12 nicht zentrifugiert wurde und nicht analysiert werden kann. In diesem Fall wird ein Benutzer informiert.

Fig. 4 zeigt ein Laborautomatisierungssystem 200. Das Laborautomatisierungssystem 200 weist eine Aufnahmestation in Form eines Probenständers 210 auf, in welchem sich vorliegend zwei Probenröhrchen 10a, 10b befinden. In den Probenständer 210 können manuell Probenröhrchen 10, 10a, 10b eingebracht werden, welche analysiert werden sollen.

Das Laborautomatisierungssystem weist ferner eine Zentrifuge 230, in welche zwei Probenröhrchen 10c, 10d eingebracht sind, auf. Mittels der Zentrifuge 230, welche eine gewöhnliche bekannte Laborzentrifuge sein kann, können Proben, welche sich in den Probenröhrchen 10, 10c, 10d befinden, mittels Fliehkraft getrennt werden.

Zwischen dem Probenständer 210 und der Zentrifuge 230 ist ein Roboter 220 vorgesehen, welcher Probenröhrchen 10a, 10b aus dem Probenständer 210 herausnehmen und in die Zentrifuge 230 einsetzen kann. Somit kann das Bestücken der Zentrifuge 230 vollautomatisiert erfolgen.

Das Laborautomatisierungssystem weist ferner ein Analysegerät 250 auf, welches gewünschte medizinische Daten einer in den jeweiligen Probenröhrchen enthaltenen Probe ermitteln kann. Das Analysegerät 250 weist eine Vorrichtung 100 zum Analysieren von Proben wie in Fig. 2 dargestellt auf.

Zwischen der Zentrifuge 230 und dem Analysegerät 250 ist ein weiterer Roboter 240 vorgesehen, welcher dazu ausgebildet ist, Probenröhrchen 10, 10c, 10d aus der Zentrifuge 230 zu entnehmen und in die Vorrichtung 100 zum Analysieren von Proben einzuführen. Vorliegend ist in der Vorrichtung 100 ein Probenröhrchen 10e gezeigt, welches von der Vorrichtung 100 zunächst daraufhin überprüft wird, ob es zentrifugiert wurde und anschließend, bei positivem Ergebnis, analysiert wird.

Das beschriebene Laborautomatisierungssystem 200 ermöglicht eine vollständige Automatisierung der Analyse nach dem Einstecken der Probenröhrchen 10 mit den darin befindlichen Proben 12 über das Zentrifugieren bis hin zum Analysieren. Das Vorsehen der erfindungsgemäßen Vorrichtung 100 zum Analysieren von Proben, welche vor dem Analysieren überprüft, ob die Proben auch tatsächlich zentrifugiert wurden, bietet einen zusätzlichen Schutz gegen Fehlfunktionen oder bei etwaigen menschlichen Eingriffen. Beispielsweise könnte ein Mensch eine nicht zentrifugierte Probe in einem entsprechenden Probenröhrchen unmittelbar in die Vorrichtung 100 einsetzen, weil er diese besonders zügig analysieren möchte. In diesem Fall würde die Vorrichtung 100 erkennen, dass die Probe nicht zentrifugiert wurde und die Analyse verweigern.

## Patentansprüche

1. Verfahren zum automatisierten Erkennen eines Zustands aus einer vorgegebenen Menge von Zuständen einer Probe (12), insbesondere einer Blutprobe, mittels einer Vorrichtung (100), wobei die Menge von Zuständen einen nicht-gemischten Zustand und einen gemischten Zustand aufweist, und wobei sich die Probe (12) in einem sich entlang einer Längsrichtung (14) erstreckenden Probenröhrchen (10) befindet,
wobei das Verfahren folgende Schritte aufweist:
- Ermitteln zumindest einer Eigenschaft der Probe (12) an einer Mehrzahl von unterschiedlichen Positionen entlang der Längsrichtung (14),
- Ermitteln mindestens eines Phasentyps einer Phase der Probe (12) in dem Probenröhrchen (10) anhand der ermittelten Eigenschaft, und
- Ermitteln des Zustands der Probe (12) als nicht-gemischter Zustand oder gemischter Zustand in Abhängigkeit von dem ermittelten Phasentyp,
- wobei beim Schritt des Ermitteins zumindest einer Eigenschaft an einer jeweiligen Position eine erste Transmission der Probe (12) quer zur Längsrichtung (14) bei einer ersten Wellenlänge als erste Eigenschaft und eine zweite Transmission der Probe (12) quer zur Längsrichtung (14) bei einer zweiten Wellenlänge als zweite Eigenschaft ermittelt werden.

2. Verfahren nach Anspruch 1, wobei wenn die erste Transmission oberhalb eines ersten Plasmaschwellenwerts (S₁) liegt und die zweite Transmission unterhalb eines zweiten Plasmaschwellenwerts (S₂) liegt der Phasentyp Plasma (24) ermittelt wird.

3. Verfahren nach Anspruch 1 oder 2, wobei wenn die erste Transmission oberhalb eines unteren ersten Gelschwellenwerts (G_{1U}) und unterhalb eines oberen ersten Gelschwellenwerts (G_{1O}) liegt und die zweite Transmission oberhalb eines unteren zweiten Gelschwellenwerts (G_{2U}) und unterhalb eines oberen zweiten Gelschwellenwerts (G_{2O}) liegt der Phasentyp Gel (22) ermittelt wird.

4. Verfahren nach einem der Ansprüche 1 bis 3, wobei wenn die erste Transmission unterhalb eines ersten Blutkuchenschwellenwerts (B₁) liegt und die zweite Transmission unterhalb eines zweiten Blutkuchenschwellenwerts (B₂) liegt der Phasentyp Blutkuchen (26) ermittelt wird.

5. Verfahren nach einem der Ansprüche 1 bis 4, wobei wenn die erste Transmission oberhalb eines unteren ersten Vollblutschwellenwerts (V_{1U}) und unterhalb eines oberen ersten Vollblutschwellenwerts (V_{1O}) liegt und die zweite Transmission oberhalb eines unteren zweiten Vollblutschwellenwerts (V_{2U}) und unterhalb eines oberen zweiten Vollblutschwellenwerts (V_{2O}) liegt der Phasentyp Vollblut (20) ermittelt wird.

6. Verfahren nach einem der vorhergehenden Ansprüche, wobei bei Ermittlung eines Phasentyps Gel (22) an einer Position zwischen einem geschlossenen Ende des Probenröhrchens (10) und einer oberen Gelposition ein gemischter Zustand ermittelt wird.

7. Verfahren nach einem der vorhergehenden Ansprüche, wobei bei Ermittlung eines Phasentyps Vollblut (20) ein gemischter Zustand ermittelt wird.

8. Verfahren nach einem der vorhergehenden Ansprüche, wobei bei Ermittlung zumindest dreier fester oder flüssiger Phasen unterschiedlicher Typen ein nicht-gemischter Zustand ermittelt wird.

9. Verfahren nach einem der vorhergehenden Ansprüche, wobei bei Ermittlung eines Phasentyps Blutkuchen (26) eine Länge der Phase des Phasentyps Blutkuchen (26) entlang der Längsrichtung (14) ausgehend von einem geschlossenen Ende des Probenröhrchens (10) und ferner eine Länge aller festen oder flüssigen Phasen (20, 22, 24, 26) im Probenröhrchen (10) ausgehend von dem geschlossenen Ende des Probenröhrchens (10) ermittelt werden, und anschließend ein Verhältnis der Länge der Phase des Phasentyps Blutkuchen (26) zur Länge aller festen oder flüssigen Phasen (20, 22, 24, 26) im Probenröhrchen (10) berechnet wird, und wobei ein nicht-gemischter Zustand ermittelt wird, wenn das Verhältnis unterhalb eines Trennungsschwellenwerts liegt, und wobei ein gemischter Zustand ermittelt wird, wenn das Verhältnis oberhalb des Trennungsschwellenwerts liegt.

10. Verfahren nach einem der vorhergehenden Ansprüche, wobei bei Ermittlung eines Phasentyps Gel (22), wenn die Phase Gel (22) um einen Mindestabstand von einem geschlossenen Ende des Probenröhrchens (10) beabstandet ist, ein nicht-gemischter Zustand ermittelt wird.

11. Verfahren nach einem der vorhergehenden Ansprüche, wobei die zumindest eine Eigenschaft der Probe (12) entlang der Längsrichtung (14) kontinuierlich ermittelt wird.

12. Vorrichtung (100) zum Analysieren von Proben, insbesondere Blutproben, welche in Probenröhrchen (10) enthalten sind, aufweisend
- eine Vorrichtung (100) zum Ermitteln zumindest einer Eigenschaft der Probe (12) an einer Mehrzahl von unterschiedlichen Positionen entlang einer Längsrichtung (14) des Probenröhrchens (10), und
- eine elektronische Steuerungsvorrichtung (110), welche Prozessormittel und diesen zugeordnete Speichermittel aufweist, wobei in den Speichermitteln Instruktionen gespeichert sind, bei deren Ausführung durch die Prozessormittel die Vorrichtung (100) ein Verfahren nach einem der vorhergehenden Ansprüche ausführt.

13. Vorrichtung (100) nach Anspruch 12, aufweisend:
- zumindest eine erste Lichtquelle (122) und eine zweite Lichtquelle (124), wobei die erste Lichtquelle (122) im Betrieb Licht mit einer ersten Wellenlänge zwischen 400 nm und 1200 nm emittiert und die zweite Lichtquelle (124) im Betrieb Licht mit einer zweiten Wellenlänge zwischen 1300 nm und 1700 nm emittiert, und
- mindestens einen Detektor (140) zur Detektion des von der ersten Lichtquelle (122) emittierten Lichts und zur Detektion des von der zweiten Lichtquelle (124) emittierten Lichts jeweils nach Durchgang durch das Probenröhrchen (10) quer zur Längsrichtung (14) an einer Position entlang der Längsrichtung (14),
- wobei die erste und die zweite Lichtquelle (122, 124) und/oder der mindestens eine Detektor (140) in Längsrichtung (14) des Probenröhrchens (10) verfahrbar angeordnet sind.

14. Laborautomatisierungssystem (200), aufweisend
- eine Aufnahmestation (210) für Probenröhrchen,
- eine Zentrifuge (230),
- Mittel (220) zum Verbringen von Probenröhrchen (10) aus der Aufnahmestation (210) in die Zentrifuge (230),
- eine Vorrichtung (100) zum Analysieren von Proben nach Anspruch 12 oder 13, sowie
- Mittel (240) zum Verbringen von Probenröhrchen (10) aus der Zentrifuge (230) in die Vorrichtung (100) zum Analysieren von Proben,
- wobei die Steuerungsvorrichtung (110) der Vorrichtung (100) zum Analysieren von Proben ausgebildet ist, das Verfahren nach einem der Ansprüche 1 bis 11 vor weiteren Analyseschritten durchzuführen und für den Fall, dass dabei ein gemischter Zustand ermittelt wird, keine weiteren Analyseschritte durchzuführen und bevorzugt eine Fehlermeldung auszugeben.

## Claims

1. Method for automated identification of a state of a sample (12), in particular a blood sample, from a predetermined set of states by means of a device (100), wherein the set of states comprises an unmixed state and a mixed state, and wherein the sample (12) is situated in a sample tube (10) extending along a longitudinal direction (14), wherein the method comprises the following steps:
- determining at least one property of the sample (12) at a plurality of different positions along the longitudinal direction (14),
- determining at least one phase type of a phase of the sample (12) in the sample tube (10) on the basis of the determined property, and
- determining the state of the sample (12) as an unmixed state or as a mixed state in a manner dependent on the determined phase type,
- wherein during the step of determining at least one property at a respective position a first transmissivity of the sample (12) across the longitudinal direction (14) at a first wavelength is determined as a first property and a second transmissivity of the sample (12) across the longitudinal direction (14) at a second wavelength is determined as a second property.

2. Method according to claim 1, wherein the plasma (24) phase type is determined if the first transmissivity lies above a first plasma threshold (S₁) and the second transmissivity lies below a second plasma threshold (S₂).

3. Method according to claim 1 or 2, wherein the gel (22) phase type is determined if the first transmissivity lies above a lower first gel threshold (G_{1U}) and below an upper first gel threshold (G_{1O}) and the second transmissivity lies above a lower second gel threshold (G_{2U}) and below an upper second gel threshold (G_{2O}).

4. Method according to any of claims 1 to 3, wherein the coagulum (26) phase type is determined if the first transmissivity lies below a first coagulum threshold (B₁) and the second transmissivity lies below a second coagulum threshold (B₂).

5. Method according to any of claims 1 to 4, wherein the whole blood (20) phase type is determined if the first transmissivity lies above a lower first whole blood threshold (V_{1U}) and below an upper first whole blood threshold (V_{1O}) and the second transmissivity lies above a lower second whole blood threshold (V_{2U}) and below an upper second whole blood threshold (V_{2O}).

6. Method according to any of the preceding claims, wherein a mixed state is determined if a gel (22) phase type is determined at a position between a closed end of the sample tube (10) and an upper gel position.

7. Method according to any of the preceding claims, wherein a mixed state is determined if a whole blood (20) phase type is determined.

8. Method according to any of the preceding claims, wherein an unmixed state is determined if at least three solid or liquid phases of different types are determined.

9. Method according to any of the preceding claims, wherein, if a coagulum (26) phase type is determined, a length of the phase of the coagulum (26) phase type is determined along the longitudinal direction (14), proceeding from a closed end of the sample tube (10), and, furthermore, a length of all solid or liquid phases (20, 22, 24, 26) in the sample tube (10) is determined, proceeding from the closed end of the sample tube (10), and a ratio between the length of the phase of the coagulum (26) phase type and the length of all solid or liquid phases (20, 22, 24, 26) in the sample tube (10) is thereupon calculated, and wherein an unmixed state is determined if the ratio lies below a separation threshold, and wherein a mixed state is determined if the ratio lies above the separation threshold.

10. Method according to any of the preceding claims, wherein, if a gel (22) phase type is determined, an unmixed state is determined if the gel (22) phase is spaced from a closed end of the sample tube (10) by a minimum distance.

11. Method according to any of the preceding claims, wherein the at least one property of the sample (12) is determined continuously along the longitudinal direction (14).

12. Device (100) for analyzing samples, in particular blood samples, which are contained in sample tubes (10), comprising
- a device (100) for determining at least one property of the sample (12) at a plurality of different positions along a longitudinal direction (14) of the sample tube (10), and
- an electronic control device (110) which comprises processing means and storage means assigned thereto, wherein instructions are stored in the storage means which, when executed by the processing means, causes the device (100) to carry out a method according to any of the preceding claims.

13. Device (100) according to claim 12, comprising:
- at least one first light source (122) and one second light source (124), wherein the first light source (122) emits light with a first wavelength between 400 nm and 1200 nm during operation and the second light source (124) emits light with a second wavelength between 1300 nm and 1700 nm during operation, and
- at least one detector (140) for detecting the light emitted by the first light source (122) and for detecting the light emitted by the second light source (124), in each case after passage through the sample tube (10) across the longitudinal direction (14) at a position along the longitudinal direction (14),
- wherein the first and the second light source (122, 124) and/or the at least one detector (140) is/are arranged in a displaceable manner in the longitudinal direction (14) of the sample tube (10).

14. Laboratory automation system (200), comprising
- a receiving station (210) for sample tubes,
- a centrifuge (230),
- means (220) for moving sample tubes (10) from the receiving station (210) into the centrifuge (230),
- a device (100) for analyzing samples according to claim 12 or 13, and
- means (240) for moving sample tubes (10) from the centrifuge (230) into the device (100) for analyzing samples,
- wherein the control device (110) of the device (100) for analyzing samples is embodied to perform the method according to any of claims 1 to 11 prior to further analysis steps and not to perform any more analysis steps, and preferably output an error message, in the case where a mixed state is determined in the process.

## Revendications

1. Procédé de reconnaissance automatisée d'un état parmi un nombre prédéfini d'états d'un échantillon (12), notamment un échantillon de sang, au moyen d'un dispositif (100), le nombre d'états incluant un état non mélangé et un état mélangé, et l'échantillon (12) se trouvant dans un tube à essai (10) qui s'étend dans une direction longitudinale (14),
le procédé comprenant les étapes suivantes :
- détermination d'au moins une propriété de l'échantillon (12) au niveau d'une pluralité de positions différentes le long de la direction longitudinale (14),
- détermination d'au moins un type de phase d'une phase de l'échantillon (12) dans le tube à essai (10) au moyen de la propriété déterminée, et
- détermination de l'état de l'échantillon (12) comme un état non mélangé ou un état mélangé en fonction du type de phase déterminé,
- lors de l'étape de détermination d'au moins une propriété au niveau d'une position correspondante, une première transmission de l'échantillon (12) transversalement à la direction longitudinale (14) à une première longueur d'onde est déterminée en tant que première propriété et une deuxième transmission de l'échantillon (12) transversalement à la direction longitudinale (14) à une deuxième longueur d'onde en tant que deuxième propriété.

2. Procédé selon la revendication 1, le type de phase plasma (24) étant déterminé lorsque la première transmission est supérieure à une première valeur de seuil de plasma (S₁) et la deuxième transmission est inférieure à une deuxième valeur de seuil de plasma (S₂).

3. Procédé selon la revendication 1 ou 2, le type de phase gel (22) étant déterminé lorsque la première transmission est supérieure à une première valeur de seuil de gel basse (G_{1U}) et inférieure à une première valeur de seuil de gel haute (G_{1O}) et la deuxième transmission est supérieure à une deuxième valeur de seuil de gel basse (G_{2U}) et inférieure à une deuxième valeur de seuil de gel haute (G_{2O}).

4. Procédé selon l'une des revendications 1 à 3, le type de phase sang coagulé (26) étant déterminé lorsque la première transmission est inférieure à une première valeur de seuil de sang coagulé (B₁) et la deuxième transmission est inférieure à une deuxième valeur de seuil de sang coagulé (B₂).

5. Procédé selon l'une des revendications 1 à 4, le type de phase sang total (20) étant déterminé lorsque la première transmission est supérieure à une première valeur de seuil de sang total basse (V_{1U}) et inférieure à une première valeur de seuil de sang total haute (V_{1O}) et la deuxième transmission est supérieure à une deuxième valeur de seuil de sang total basse (V_{2U}) et inférieure à une deuxième valeur de seuil de sang total haute (V_{2O}).

6. Procédé selon l'une des revendications précédentes, selon lequel, dans le cas de la détermination d'un type de phase gel (22), un état mélangé est déterminé à une position entre une extrémité fermée du tube à essai (10) et une position de gel haute.

7. Procédé selon l'une des revendications précédentes, un état mélangé étant déterminé dans le cas de la détermination d'un type de phase sang total (20).

8. Procédé selon l'une des revendications précédentes, un état non mélangé étant déterminé dans le cas de la détermination d'au moins trois phases solides ou liquides de types différents.

9. Procédé selon l'une des revendications précédentes, selon lequel, dans le cas de la détermination d'un type de phase sang coagulé (26), une longueur de la phase du type de phase sang coagulé (26) le long de la direction longitudinale (14) à partir d'une extrémité fermée du tube à essai (10) et, de plus, une longueur de toutes les phases solides ou liquides (20, 22, 24, 26) dans le tube à essai (10) à partir d'une extrémité fermée du tube à essai (10) sont déterminées, et ensuite un rapport entre la longueur de la phase du type de phase sang coagulé (26) et la longueur de toutes les phases solides ou liquides (20, 22, 24, 26) dans le tube à essai (10) est calculé, et un état non mélangé étant déterminé lorsque le rapport est inférieur à une valeur de seuil de séparation et un état mélangé étant déterminé lorsque le rapport est supérieur à la valeur de seuil de séparation.

10. Procédé selon l'une des revendications précédentes, selon lequel, dans le cas de la détermination d'un type de phase gel (22), un état non mélangé est déterminé lorsque la phase gel (22) est espacée d'un écart minimum d'une extrémité fermée du tube à essai (10).

11. Procédé selon l'une des revendications précédentes, l'au moins une propriété de l'échantillon (12) étant déterminée continuellement le long de la direction longitudinale (14).

12. Dispositif (100) pour analyser des échantillons, notamment des échantillons de sang qui sont contenus dans des tubes à essai (10), comprenant
- un dispositif (100) destiné à déterminer au moins une propriété de l'échantillon (12) au niveau d'une pluralité de positions différentes le long de direction longitudinale (14) du tube à essai (10), et
- un dispositif de commande électronique (110), lequel possède des moyens de processeur et des moyens de mémoire associés à ceux-ci, des instructions étant enregistrées dans les moyens de mémoire qui, lorsqu'elles sont exécutées par les moyens de processeur, amènent le dispositif (100) à mettre en oeuvre un procédé selon l'une des revendications précédentes.

13. Dispositif (100) selon la revendication 12, comprenant :
- au moins une première source de lumière (122) et une deuxième source de lumière (124), la première source de lumière (122) émettant en fonctionnement une lumière ayant une première longueur d'onde entre 400 nm et 1200 nm et la deuxième source de lumière (124) émettant en fonctionnement une lumière ayant une deuxième longueur d'onde entre 1300 nm et 1700 nm, et
- au moins un détecteur (140) destiné à détecter la lumière émise par la première source de lumière (122) et à détecter la lumière émise par la deuxième source de lumière (124) respectivement après le passage à travers le tube à essai (10) transversalement à la direction longitudinale (14) à une position le long de la direction longitudinale (14),
- la première et la deuxième source de lumière (122, 124) et/ou l'au moins un détecteur (140) étant montés mobiles dans la direction longitudinale (14) du tube à essai (10).

14. Système d'automatisation de laboratoire (200), comprenant :
- une station d'accueil (210) pour des tubes à essai,
- une centrifugeuse (230),
- des moyens (220) destinés à transférer des tubes à essai (10) de la station d'accueil (210) dans la centrifugeuse (230),
- un dispositif (100) destiné à analyser les échantillons selon la revendication 12 ou 13, ainsi que
- des moyens (240) destinés à transférer les tubes à essai (10) de la centrifugeuse (230) dans le dispositif (100) destiné à analyser les échantillons,
- le dispositif de commande (110) du dispositif (100) destiné à analyser les échantillons étant configuré pour mettre en oeuvre le procédé selon l'une des revendications 1 à 11 avant des étapes d'analyse supplémentaires et, dans le cas où un état mélangé est ici déterminé, pour n'exécuter aucune étape d'analyse supplémentaire et de préférence délivrer un message d'erreur.
